# NEUE EUROPÄISCHE PATENTSCHRIFT

(11) **EP 0 167 909 B2**
(45) Veröffentlichungstag und Bekanntmachung des Hinweises auf die Entscheidung über den Einspruch: **19.10.1994**
(45) Hinweis auf die Patenterteilung: 08.01.1992
(21) Anmeldenummer: 85107733.9
(22) Anmeldetag: 22.06.1985
(51) Int. Cl.: A61K 31/44, A61K 9/20, A61K 9/16

(54) **Feste Arzneizubereitungen mit Dihydropyridin und Verfahren zu ihrer Herstellung**
Solid pharmaceutical preparations containing dihydropyridine, and process for their manufacture
Préparations pharmaceutiques solides contenant dihydropyridine et procédé pour leur préparation

(30) Priorität: 04.07.1984 DE 3424553
(43) Veröffentlichungstag der Anmeldung: 15.01.1986
(73) Patentinhaber: BAYER AG, 51368 Leverkusen (DE)
(72) Erfinder: Schmidt, Wolfgang, D-5000 Köln 80 (DE); Luchtenberg, Helmut, Dr., D-5216 Niederkassel 6 (DE); Porges, Eduard, Dr., D-5000 Köln 71 (DE)

(56) Entgegenhaltungen:
- EP-A- 0 001 247
- EP-A- 0 004 650
- EP-A- 0 078 430
- EP-A- 0 143 857
- DE-A- 2 822 882
- GB-A- 2 139 892
- JOURNAL OF PHARMACEUTICAL SCIENCES, Band 62, Nr. 1, Januar 1973, Seiten 43-49, Washington, US; S.S. KORNBLUM et al.: "A new tablet disintegrating agent: cross-linked polyvinylpyrrolidone"
- Pharm. Ind. 40, Nr. 11a (1978), S. 1255-63

## Beschreibung

Die vorliegende Erfindung betrifft spezielle schnell resorbierbare feste Arzneizubereitungen die Nimodipin, Polyvinylpyrrolidon und quervernetztes Polyvinylpyrrolidon enthalten sowie Verfahren zu ihrer Herstellung.

Dihydropyridine und ihre calciumantagonistische Wirkung sind bekannt (vgl. britisches Patent 1 173 862 und britisches Patent 1 358 951). Viele Vertreter dieser Stoffklasse sind sehr lichtempfindlich und besitzen eine extrem niedere Löslichkeit in wäßrigen Medien. Die Wasserlöslichkeit von Nifedipin beträgt z.B. nur 10 mg pro Liter und die Wasserlöslichkeit von Nimodipin beträgt nur 2 mg pro Liter. Aufgrund dieser speziellen Eigenschaften treten bei der Zubereitung von galenischen Spezialitäten eine Reihe von Schwierigkeiten auf wie aus zahlreichen Patentanmeldungen für spezielle Formulierungen dieser Wirkstoffe ersichtlich ist.

Das US-Patent 3 784 684 betrifft z.B. spezielle Gelatine-Beißkapseln, die Nifedipin in gelöster Form enthalten um die Koronarwirkung von Nifedipin vorteilhaft zu nutzen. In dem britischen Patent 1 456 618 werden feste Arzneizubereitungen beschrieben und beansprucht die eine gute Bioverfügbarkeit der Dihydropyridine gewährleisten sollen.

In der DT-OS 2 822 882 werden ebenfalls feste Arzneizubereitungen beschrieben, bei denen durch den Einsatz bestimmter Lösungsvermittler und oberflächenaktiver Substanzen die Schwerlöslichkeit von Dihydropyridinen kompensiert werden soll.

Auch in der EP-OS 1 247 soll die Resorbierbarkeit von Nifedipin durch die Verwendung von Polyethylenglykol (PEG) und bestimmter poröser Trägersubstanzen verbessert werden. In dieser Anmeldung wird auch beschrieben, daß die schlechte Löslichkeit von Nifedipin ausgeglichen werden kann, durch die Bildung von Copräzipitaten aus Nifedipin und Polyvinylpyrrolidon (PVP), in der das Nifedipin in amorpher Form als feste Lösung vorliegt.

Diese Copräzipitate werden hergestellt durch Lösen von Nifedipin und PVP in organischen Lösungsmitteln mit anschließender Verdampfung des Lösungsmittels um eine glasige Masse zu erhalten (vgl. DT-OS 2 822 882). Eine solche Verdampfung des organischen Lösungsmittels läßt sich technisch nur mit großem Aufwand durchführen, da die PVP-Masse die organischen Lösungsmittel stark bindet und kurz vor dem Trocknen sehr viskos wird. Es entsteht eine voluminöse schaumige Masse, die kurz vor Ende der Trocknung sehr zäh ist, nicht mehr gerührt werden kann und nur schwierig weiterverarbeitet werden kann. Eine vollständige Entfernung des Lösungsmittels aus dem Copräzipitat ist praktisch nicht möglich. Ein weiterer Nachteil bei der Verwendung Von Nifedipin-PVP-Copräzipitat bei der Tablettenherstellung ist die Tatsache, daß dieses Copräzipitat sich mit anderen Hilfsstoffen Zwar mischen läßt, aber nicht mit wäßrigen Lösungen granuliert werden kann. Eine solche einfache Mischung zwischen dem PVP-Copräzipitat und anderen Hilfsstoffen neigt jedoch zu Entmischungen bei der weiteren maschinellen Verarbeitung, z.B. zu Tabletten oder beim Abfüllen in Kapseln. Dies kann letztlich zu Arzneizubereitungen mit sehr unterschiedlichem Wirkstoffgehalt in den einzelnen Tabletten oder Kapseln führen (mangelnde "Content Uniformity"), was bei einer hochwirksamen Substanz wie Nifedipin äußerst unerwünscht ist. Darüber hinaus sind die zur Auswahl stehenden zusätzlichen Hilfsstoffe sehr begrenzt, insbesondere für die Tablettenherstellung weil PVP gleichzeitig als Bindemittel wirkt und durch die Anwesenheit von größeren Mengen von PVP (30 bis 100 mg pro Tablette oder Kapsel) der Zerfall der Tabletten bzw. Kapseln verhindert wird.

Gemäß der DE-OS 3 142 853 werden die bisher genannten Nachteile dadurch behoben, daß eine größere Menge an Hilfsstoffen zur Absorption der Lösung Dihydropyridin, Polyvinylpyrrolidon/Aceton verwendet wird. Solche Formulierungen sind akzeptabel, wenn die Wirkstoffe nur in niedrigen Dosierungen eingesetzt werden sollen.

Wenn jedoch höhere Dosierungen, z. B. 30 oder 100 mg Wirkstoff, verwendet werden sollen, besitzt diese Methode den Nachteil, daß das Verhältnis Wirkstoff zu Hilfsstoff konstant bleiben muß und somit sehr große Zubereitungsformen erhalten werden, die von Patienten schlecht einzunehmen sind (z. B. 2 g schwere Tabletten).

Aus Pharm. Ind. 40, Nr. 11 a (1978), Seite 1255-1263 ist bereits bekannt. quervernetztes Polyvinylpyrrolidon (PVPP; Polyplasdone XL) in geringen Mengen als Zerfalls- bzw. als Bindemittel in Arzneizubereitungen einzusetzen. Einen Hinweis auf die Verwendung von PVPP als Trägermaterial bzw. auf seine Verwendung zur Erteichung einer stabilen Übersättigung für schwer lösliche Wirkstoffe ist aus dieser Literatur nicht zu entnehmen. Vielmehr wird dort ausdrücklich gesagt, daß PVPP nur in wenigen mg zugesetzt wird und daß das Prinzip der Übersättigung auf die Kieselsäure-Matrix-Fixierung zurückzuführen ist. Auf Seite 1260 wird in der rechten Spalte auf die DE-OS 2 634 004 hingewiesen, wonach PVPP auch als Trägersubstanz eingesetzt werden kann und dann die Lösungsgeschwindigkeit und auch die Übersättigung steigern können soll. Im Unterschied zu den enfindungsgemäßen Zubereitungen enthalten die dort beschriebenen Formulierungen kein PVP. Es hat sich gezeigt, daß eine Übertragung der dort gegebenen technischen Lehre auf Nimodipin nicht zum Erfolg führt. Um Nimodipin in entsprechend kleinen Zubereitungsformen mit ausreichender Bioverfügbarkeit zu erhalten, ist es zwingend erforderlich, geringe Mengen von PVP zuzugeben.

Die vorliegende Erfindung betrifft neue feste Dihydropyridinzubereitungen in Form von Granulat mit schneller Resorbierbarkeit und gleichmäßigem Wirkstoffgehalt, deren relative Standardabweichung höchstens 1,5 % beträgt, enthaltend 1 Gew.-Teil Nimodipin als Wirkstoff, 0,1 bis 1,0 Gew.-Teile Polyvinylpoyrrolidon mit einem mittleren Molekulargewicht von 15000 bis 50000 und als Stabilsator 2 bis 6 Gew.-Teile quervernetztes unlösliches Polyvinylpyrrolidon (PVPP) als Adsorbant und Sprengmittel sowie gegebenenfalls weitere übliche Hilfsstoffe.

Vorzugsweise seien Arzneizubereitungen genannt, die Nimodipin in einer Dosierung von 1 bis 100 mg, insbesondere 5 bis 70 mg enthalten.

Als Hilfsstoffe seien vorzugsweise genannt: Netzmittel wie z.B. Natriumlaurylsulfat und Tween(^{R}), wobei diese Netzmittel in maximaler Menge von 1 Gew.-Teil eingesetzt werden. Weiterhin seien genannt: Schmiermittel wie z.B. Magnesiumstearat. Auch die Anwesenheit von Füllstoffen wie Zucker, z.B. Milchzucker, Mannit, Glycocol, Calciumcarbonat, Maisstärke und Avicel kann vorteilhaft sein. Gegebenenfalls können die Tabletten auch noch Sprengmittel enthalten oder nachträglich mit einer üblichen Lackierung versehen werden um eine so fortige Löslichkeit, eine Retardierung oder eine Magensattresistenz der erfindungsgemäßen Formulierung zu erreichen. Weiterhin lassen sich die erfindungsgemäßen Formulierungen, insbesondere die Granulate mit anderen galenischen Formen, die gleiche oder verschiedene Wirkstoffe enthalten. kombinieren.

Die Herstellung der erfindungsgemäßen festen Zubereitung erfolgt indem man 1 Gew.-Teil Nimodipin und 0,01 bis 1,5 Gew.-Teile PVP in einer entsprechenden Menge organischer Lösungsmittel, in denen sich die beiden festen Bestandteile lösen lassen, auflöst, gegebenenfalls ein Netzmittel darin suspendiert oder löst, und diese Lösung mit 1 bis 10 Gew.-Teilen PVPP granuliert und das erhaltene Granulat gegebenenfalls nach Zusatz weiterer Hilfsstoffe zu festen Arzneizubereitungen weiterverarbeitet.

Als feste Arzneizubereitungen seien vorzugsweise genannt: Tabletten, Pillen, Dragees, Granulate, Pulver, Kapseln, Sachets und Pellets.

Als organische Lösungsmittel seien vorzugsweise genannt: Ethanol, Methanol, Aceton, Methylenchlorid und Chloroform sowie Gemische dieser Lösungsmittel. Sie werden vorzugsweise in Mengen von 6 bis 50, insbesondere von 8 bis 20 Gew.-Teilen bezogen auf das Dihydropyridin eingesetzt.

Es war nicht vorhersehbar, daß die so erhaltenen Arzneizubereitungen eine sehr gute Bioverfügbarkeit gleichzeitig eine gute Content-Uniformity beibehalten. Die durch das erfindungsgemäße Verfahren erhaltenen Granulate, bestehend aus dem mit Nimodipin-PVP-Lösung benetzten PVPP, lassen sich problemlos trocknen, sieben, weiterverarbeiten, mit anderen Hilfsstoffen vermischen und zu Tabletten pressen. Die Bioverfügbarkeit der erfindungsgemäßen Zubereitungen ist in ihrer Schnelligkeit und in ihrer Höhe den bisher bekannten Zubereitungen mindestens gleichwertig. Sie besitzt darüber hinaus den Vorteil, daß das Verfahren mit sehr geringer Energie und in kurzer Zeit durchgeführt werden kann. Insbesondere seien die leichte Trocknung und die damit verbundene kürzere Trocknungszeit (maximal 20 % der herkömmlichen Trocknungszeit) hervorgehoben. Die erhaltenen festen Zubereitungen sind sehr klein und können trotz hohen Wirkstoffsgehalt ohne Mühe vom Patienten eingenommen werden.

Die Trocknung des feuchten Granulats geht durch den geringen PVP-Anteil in wesentlich kürzerer Zeit von statten (maximal 20 % der herkömmlichen Trocknungszeit). Aus dem gleichen Grunde treten bei der Trocknung keine Verklumpungen auf.

Durch die vorliegende Erfindung werden, im Gegensatz zu bekannten Formulierungen, die durch Granulation mit organischen Lösungsmitteln hergestellt werden, die Lösungsmittelrestgehalte so stark reduziert, daß sie unter 0,05 % liegen und so praktisch nach herkömmlichen Analysenmethoden nicht mehr nachgewiesen werden können. Diese Reduzierung von Lösungsmittelrestgehalten ist aus toxikologischer Sicht, insbesondere bei Langzeitbehandlungen, vorteilhaft.

So hergestellte Tabletten besitzen Zerfallszeiten die unter 5 Minuten liegen. Durch das erfindungsgemäße Verfahren werden Hilfsstoffe eingespart und damit nicht nur die orale Applikation erleichtert sondern gleichzeitig auch die Kosten bei der Herstellung gesenkt.

### Beispiel 1

30 g Nimodipine, 17 g PVP 25 werden in 500 g Aceton gelöst und 0.5 g Natriumlaurylsulfat in der Lösung suspendiert. In einem Granuliergerät wird 107 g quervernetztes PVPP mit dieser Lösung granuliert. Das Granulat wird getrocknet und gesiebt. Nach Zumischung von 1,5 g Magnesiumstearat wird das Granulat in Tabletten von 156 mg Gewicht verpreßt, enthaltend jeweils 30 mg Nimodipin. Die Tabletten zeichnen sich durch geringes Gewicht, gute Zerfallszeiten. und vorteilhafte Freisetzung aus.

### Beispiel 2

Das in Beispiel 1 erhaltene Granulat wird zu Tabletten mit 520 mg Gewicht verpreßt. enthaltend jeweils 100 mg Nimodipin.

### Beispiel 3

30 g Nimodipin, 4 g PVP 25 werden in 800 g Aceton gelöst und 0,5 g Natriumlaurylsulfat in der Lösung suspendiert. In einem Granuliergerät wird 180 g quervernetztes PVPP mit dieser Lösung granuliert. Das Granulat wird getrocknet, gesiebt und nach Zumischung von 2,0 g Magnesiumsulfat zu Tabletten von 216 mg verpreßt, enthaltend jeweils 30 mg Nimodipin.

### Beispiel 4

Das Granulat aus Beispiel 1 wird gewalzt oder brikettiert und das nach dem zersieben erhaltene Granulat in Kapseln oder Sachets abgefüllt.

### Beispiel 5

Das in Beispiel 1 beschriebene Lösungsmittel Aceton wird ersetzt durch die gleiche Menge Methylenchlorid oder Chloroform oder einem Gemisch derselben.

## Patentansprüche

1. Schnell resorbierbares Granulat ohne Lösungsmittelrestgehalt mit einer maximalen Standardabweichung des Wirkstoffgehaltes von 1.5 %. enthaltend 1 Gewichtsteil Nimodipin, 0,1 - 1,0 Gewichtsteil Polyvinylpyrrolidon mit einem mittleren Molekulargewicht von 15.000 - 50.000 und 2 bis 6 Gewichtsteile quervernetztes unlösliches Polyvinylpyrrolidon (PVPP) sowie gegebenenfalls weitere Hilfsstoffe.

2. Granulat gemäß Anspruch 1 enthaltend als weitere Hilfsstoffe bis zu 1 Gewichtsteil Netzmittel aus der Gruppe Natriumlaurylsulfat oder Tween.

3. Verfahren zur Herstellung von Granulaten gemäß Anspruch 1, dadurch gekennzeichnet, daß man 1 Gewichtsteil Nimodipin und 0,1 bis 1,0 Gewichtsteile Polyvinylpyrrolidon in 6 bis 50 Gewichtsteilen eines organischen Lösungsmittels auflöst, gegebenenfalls ein übliches Netzmittel zugibt und diese Lösung mit 2 bis 6 Gewichtsteilen PVPP granuliert und anschließend nach üblichen Methoden das Lösungsmittel entfernt bis zu einem Restgehalt unter 0,05 %.

4. Verfahren gemäß Anspruch 3, dadurch gekennzeichnet, daß man als organische Lösungsmittel Methanol, Ethanol, Aceton, Methylenchlorid oder Chloroform oder Gemische dieser Lösungsmittel verwendet.

5. Verwendung von Granulaten gemäß Anspruch 1 bei der Herstellung von festen schnell resorbierbaren Arzneizubereitungen in Form von Granulaten, Pulvern, Tabletten oder Dragees mit einem Wirkstoffgehalt von 5 bis 70 mg pro Zubereitungsform.

## Claims

1. Rapidly absorbable granules without residual solvent, with a maximum standard deviation of the active compound content of 1.5%, containing 1 part by weight of nimodipine, 0.1 to 1.0 part by weight of polyvinylpyrrolidone with an average molecular weight of 15,000 to 50,000, and 2 to 6 parts by weight of crosslinked insoluble polyvinylpyrrolidone (PVPP) and, if appropriate, further auxiliaries.

2. Granules according to Claim 1, containing as further auxiliaries up to 1 part by weight of wetting agent from the group comprising sodium lauryl sulphate or Tween.

3. Process for the preparation of granules according to Claim 1, characterised in that 1 part by weight of nimodipine and 0.1 to 1.0 part by weight of polyvinylpyrrolidone are dissolved in 6 to 50 parts by weight of an organic solvent, a conventional wetting agent is added if appropriate, and this solution is granulated with 2 to 6 parts by weight of PVPP, and the solvent is then removed, using conventional methods, to give a residual content below 0.05%.

4. Process according to Claim 3, characterised in that methanol, ethanol, acetone, methylene chloride or chloroform or mixtures of these solvents are used as the organic solvents.

5. Use of granules according to Claim 1 in the preparation of solid, rapidly absorbable medical formulations in the form of granules, powders, tablets or dragees with an active compound content of 5 to 70 mg per preparation form.

## Revendications

1. Granulat rapidement résorbable, dépourvu de teneur résiduelle en solvants, présentant un écart type maximal de 1,5 % pour la teneur en principe actif, contenant 1 partie en poids de nimodipine, de 0,1 à 1,0 partie en poids de polyvinylpyrrolidone de poids moléculaire moyen compris entre 15 000 et 50 000 et 2 à 6 parties en poids de polyvinylpyrrolidone réticulée insoluble (PVPP), ainsi que d'autres adjuvants le cas échéant.

2. Granulat selon la revendication 1, contenant comme autres adjuvants jusqu'à 1 partie en poids d'agents mouillants du groupe laurylsul- fate de sodium ou Tween.

3. Procédé de préparation de granulats selon la revendication 1, caractérisé en ce qu'on dissout 1 partie en poids de nimodipine et de 0,1 à 1,0 partie en poids de polyvinylpyrrolidone dans 6 à 50 parties en poids d'un solvant organique, qu'on ajoute un agent mouillant usuel le cas échéant et qu'on granule cette solution avec 2 à 6 parties en poids de PVPP et qu'on élimine ensuite le solvant jusqu'à une teneur résiduelle inférieure à 0,05 % par des méthodes usuelles.

4. Procédé selon la revendication 3, caractérisé en ce qu'on utilise comme solvants organiques du méthanol, de l'éthanol, de l'acétone, du chlorure de méthylène ou du chloroforme ou des mélanges de ces solvants.

5. Emploi de granulats selon la revendication 1, pour la fabrication de préparations pharmaceutiques solides rapidement résorbables sous la forme de granulats, de poudres, de comprimés ou de comprimés dragéifiés ayant une teneur en principe actif comprise entre 5 et 70 mg par forme de préparation.
